# EUROPÄISCHE PATENTSCHRIFT

(11) **EP 1 721 964 B1**
(45) Veröffentlichungstag und Bekanntmachung des Hinweises auf die Patenterteilung: **21.05.2008**
(21) Anmeldenummer: 06009405.9
(22) Anmeldetag: 08.05.2006
(51) Int. Cl.: C12M 1/02, C12M 1/00

(54) **Lagervorrichtung für Laborproben mit Lagerschächten und Schüttelantrieb**
Storage device for laboratory specimens with storage towers and shaking device
Dispositif de stockage d'échantillons de laboratoire avec des tours de stockage et dispositif de vibration

(30) Priorität: 09.05.2005 CH 8132005
(43) Veröffentlichungstag der Anmeldung: 15.11.2006
(73) Patentinhaber: LICONIC AG, 9493 Mauren (LI)
(72) Erfinder: Malin, Cosmas. G, 9493 Mauren (LI)
(74) Vertreter: Blum, Rudolf Emil

(56) Entgegenhaltungen:
- EP-A- 0 569 214
- EP-A- 1 443 101
- DE-A1- 10 009 555
- DE-A1- 10 302 809

## Beschreibung

Die vorliegende Erfindung bezieht sich auf eine Lagervorrichtung für Laborproben mit Lagerschächten und Schüttelantrieb, sowie auf einen Klimaschrank mit einer derartigen Lagervorrichtung.

In automatisierten Versuchen, z.B. in der Mikrobiologie, werden Proben in sogenannten Mikrotiterplatten mit einer grossen Anzahl einzelner kleiner Zellen (Wells) gelagert. Wegen der grossen Vielzahl von Versuchen werden weiter eine grosse Anzahl Platten während eines Versuchs eingesetzt. Typischerweise sind die Platten hierbei in turmförmigen Lagerschächten untergebracht. Jeder Lagerschacht ist ausgestaltet, um mehrere Mikrotiterplatten (oder ähnliche Laborproben) übereinander aufzunehmen. Für bestimmte Anwendungen ist es notwendig, die Proben über eine bestimmte Zeit einer Schüttelbewegung auszusetzen.

Aus EP 1 443 101 ist eine Lagervorrichtung in einem Klimaschrank bekannt, welche einen Schüttelantrieb aufweist, der einen Schachtträger schüttelt, wobei auf dem Schachtträger z.B. zwei Lagerschächte angeordnet sind. Da die Lagerschächte relativ hoch sind, kann es bei der Schüttelbewegung zu unerwünschten Bewegungen insbesondere in den oberen Endbereichen der Lagerschächte kommen. Dies limitiert die Schüttelfrequenzen- und amplituden, macht einen aufwändigen Aufbau erforderlich und führt zu unerwünschtem Lärm und Vibrationen sowie zu einer höhenabhängigen Schüttelamplitude.

Es stellt sich deshalb die Aufgabe, eine Lagervorrichtung bzw. einen Klimaschrank bereitzustellen, der ein möglichst effizientes Schütteln auch hoher Lagerschächte erlaubt. Diese Aufgabe wird vom Gegenstand der unabhängigen Ansprüche gelöst.

In der Erfindung wird eine Lagervorrichtung bereitgestellt, die eine Gegengewicht-Anordnung besitzt. Diese ist mit einem oberen Endbereich des Lagerschachts (bzw. der Lagerschächte) verbunden und wird so angetrieben, dass sie auf den oberen Endbereich eine zur Schüttelbewegung gegenläufige Kraft ausübt, um so einem durch die Schüttelbewegung erzeugten Schwingen des oberen Endbereichs des Lagerschachts entgegenzuwirken. Auf diese Weise werden unerwünschte Auslenkungen im oberen Endbereich des Lagerschachts reduziert und es wird eine homogenere Schüttelbewegung erzielt.

In einer vorteilhaften Ausführung besitzt die Gegengewicht-Anordnung einen bewegten Teil, welcher gegenläufig zur Schüttelbewegung angetrieben wird und so die genannte Kraft erzeugen kann.

Die Erfindung bezieht sich auch auf einen Klimaschrank mit einer derartigen Lagervorrichtung, in welchem die Lagerschächte in kontrollierter Temperatur und ggf. in kontrollierter Gaszusammensetzung, insbesondere unter kontrollierter Feuchte, gelagert werden können.

In allen Aspekten der Erfindung können hohe Beschleunigungskräfte erzielt werden, so dass insbesondere auch kleine Flüssigkeitsmengen gut durchmischt werden können.

Weitere bevorzugte Ausführungen der Erfindung ergeben sich aus den abhängigen Ansprüchen sowie aus der folgenden Beschreibung anhand der Figuren. Dabei zeigen:
Fig. 1 einen Klimaschrank mit einem Schüttelantrieb für vertikales Schütteln,
Fig. 2 ein Detail des Schüttelantriebs von Fig.1,
Fig. 3 eine zweite Ausführung eines Klimaschranks mit einem Schüttelantrieb für vertikales Schütteln,
Fig. 4 eine dritte Ausführung der Erfindung,
Fig. 5 die Ausführung nach Fig. 4 aus einem etwas anderen Winkel gesehen,
Fig. 6 die Ausführung nach Fig. 4 aus einem weiteren Winkel gesehen, mit zusätzlicher Schutzplatte,
Fig. 7 eine Draufsicht auf die Grundplatte der Ausführung nach Fig. 4 mit den Riemenrädern, wobei der Verlauf des Riemens und die Anordnung der Lagerschächte gestrichelt eingezeichnet sind,
Fig. 8 die obere Abstützung der Gegengewicht-Anordnung
Fig. 9 eine Halteschiene für einen Lagerschacht und
Fig. 10 eine Variante zur Ausführung nach Fig. 7.

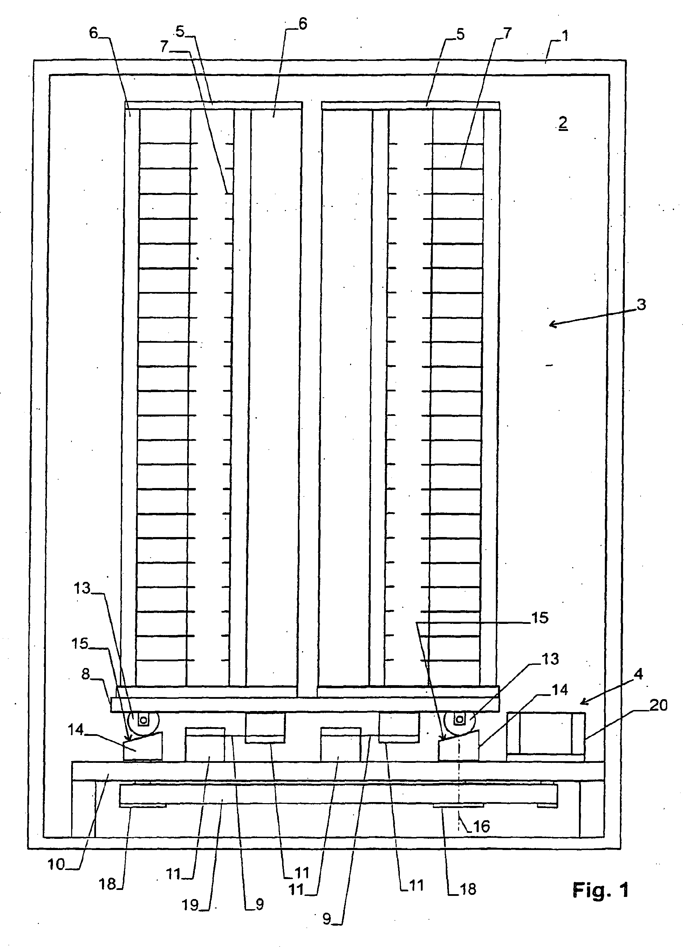

In Fig. 1 ist ein Klimaschrank 1 dargestellt, in dessen Innenraum 2 eine Lagervorrichtung 3 mit Schüttelantrieb 4 angeordnet ist. Der Klimaschrank besitzt Mittel, um in Innenraum ein kontrolliertes Klima zu erzeugen. Er kann für den manuellen und/oder automatischen Zugriff ausgelegt sein. Der in Fig. 1 dargestellte Klimaschrank entspricht beispielsweise dem in US 6 478 524 beschriebenen Gerät mit zwei V-förmig angeordneten Lagerschächten 5. Der in Fig. 1 dargestellte Schüttelantrieb 4 eignet sich jedoch auch für Lagervorrichtungen mit nur einem oder wesentlich mehr Lagerschächten. Auch kann er für Lagervorrichtungen 3 angewendet werden, die nicht in einem Klimaschrank angeordnet sind. Das Gleiche gilt auch für die weiter unten beschriebenen anderen Schüttelantriebe 4 bzw. Lagervorrichtungen 3.

Jeder Lagerschacht 5 besitzt zwei vertikale Seitenwände 6, an denen je eine Vielzahl von Auflageschienen 7 angeordnet sind. Diese bilden eine entsprechende Zahl übereinander angeordneter Lagerplätze zur Aufnahme von Laborproben. In einer besonders wichtigen Anwendung handelt es sich bei den Laborproben um Mikrotiterplatten. Diese besitzen eine Vielzahl von feinen Aufnahmevertiefungen für die Proben. Mikrotiterplatten haben in der Regel eine genormte Grösse mit einer Grundfläche von ca. 127.76 mm × 85.48 mm (je ± 0.5 mm) gemäss Standard ANSI/SBS 1-2004, 1/8/2004, "for microplates - Footprint Dimensions". Die Lagerplätze sind diesen Dimensionen angepasst.

Die Lagerschächte 5 sind auf einem gemeinsamen Schachtträger 8 angeordnet und dort in geeigneter, lösbarer Weise befestigt. Der Schachtträger 8 bietet hierfür, wie später beschrieben, z.B. eine horizontale Auflagefläche mit Befestigungsschienen.

Der Schachtträger 8 ist vertikal bewegbar und horizontal geführt gelagert. Im Ausführungsbeispiel nach Fig. 1 dienen hierzu zwei Blattfedern 9, die zwischen dem Schachtträger 8 und einem ruhenden Grundkörper 10 befestigt sind. Die Blattfedern sind horizontal angeordnet, so dass sie eine vertikale Bewegung des Schachtträgers 8 zulassen, eine horizontale Bewegung aber verhindern. Hierzu ist am Grundkörper 10 und am Schachtträger 8 für jede Blattfeder 9 je ein Befestigungsblock 11 angeordnet, wobei jede Blattfedern im Wesentlichen horizontal zwischen ihren beiden Befestigungsblöcken 11 eingespannt ist.

Zum Erzeugen der Schüttelbewegung sind z.B. vier Taumellager vorgesehen. Hierzu sind am Schachtträger vier Laufkörper 13 angeordnet, von denen jeder auf einer nicht-horizontalen Endfläche 15 eines Drehkörpers 14 des Schüttelantriebs aufliegt.

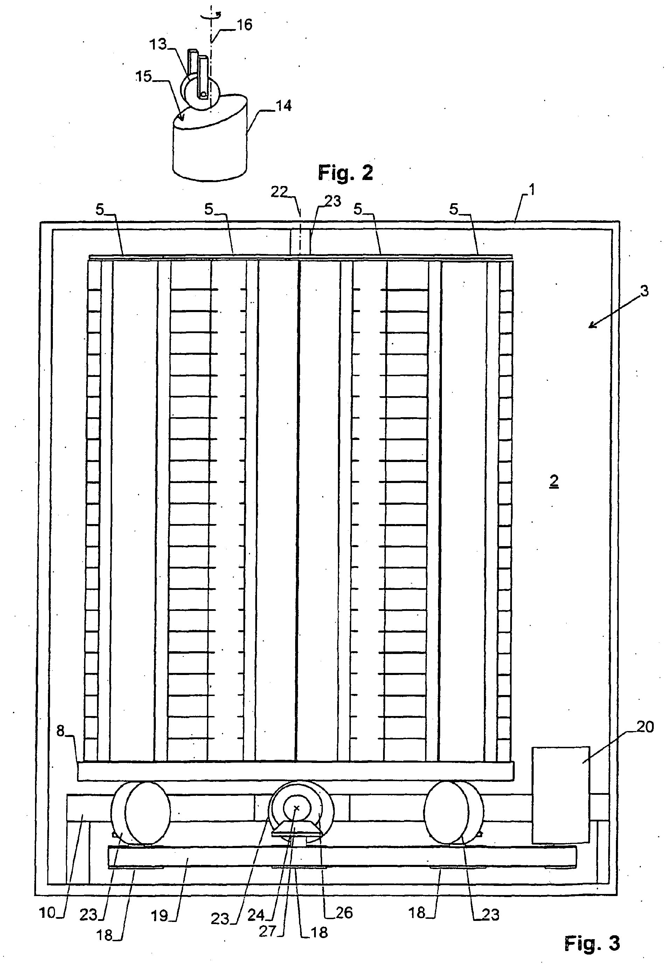

Wie im Folgenden beschrieben und insbesondere in Fig. 2 dargestellt, ist jeder Drehkörper 14 zum Drehen um eine vertikale Drehachse 16 angetrieben. Jede Endfläche 15 ist eben und geneigt zur Drehachse 16 des jeweiligen Drehkörpers 14, und der Laufkörper 13 berührt die Endfläche 15 in einem Abstand zur Drehachse.

Wird der Drehkörper 14 um die Drehachse 16 gedreht, so führt der jeweilige Laufkörper 13 relativ zum Drehkörper 14 eine Auf- und Abbewegung durch. Die Blattfedern 9 stellen dabei sicher, dass die Laufkörper 13 auch bei hohen Schüttelfrequenzen nicht von den Endflächen 15 abheben.

Wie in Fig. 1 dargestellt, sind die Drehkörper 14 im Grundkörper 10 gelagert. Jeder Drehkörper 14 ist mit einem Antriebsritzel 18 verbunden. Um die Antriebsritzel 18 ist ein Zahnriemen 19 gelegt, der von einem Motor 20 angetrieben wird. Somit ist sichergestellt, dass die Drehkörper synchron zueinander angetrieben werden, so dass sich die Laufkörper 13 im Gleichtakt bewegen.

Wie insbesondere aus Fig. 2 ersichtlich, ist in der dargestellten Ausführung der Laufkörper 13 als Rad ausgestaltet, das auf der Endfläche 15 läuft und diese im Abstand zur Drehachse 16 berührt. Durch die Verwendung eines Rads können Reibungsverluste gering gehalten werden. Es ist jedoch möglich, anstelle eines Rads auch einen Gleiter als Laufkörper 13 zu verwenden, der auf der Endfläche 15 gleitet.

Anstelle einer ebenen, geneigten Endfläche 15 kann auch eine gewellte oder gekrümmte Endfläche 15 eingesetzt werden. Ferner ist es auch denkbar, den Laufkörper 13 am Grundkörper 10 und den Drehkörper 14 (mit dem Schüttelmotor 20) am Schachtträger 8 anzuordnen.

Anstelle des in Fig. 1 und 2 dargestellten Schüttelantriebs kann auch ein anderer Antrieb eingesetzt werden, mit welchem der Schachtträger 8 in eine Auf- und Abbewegung versetzt werden kann. Vorzugsweise erfolgt diese Bewegung in rein vertikaler Richtung, damit das eingangs erwähnte, problematische seitliche Auslenken der Lagerschächte 5 ganz vermieden werden kann.

Beispielsweise kann als Schüttelantrieb auch ein direkter, elektromagnetischer Vibrationsantrieb vorgesehen sein.

Eine andere Variante eines Schüttelantriebs ist in Fig. 3 dargestellt.

Auch hier handelt es sich um eine Lagervorrichtung 3 in einem Klimaschrank 1. In dieser Ausführung besitzt die Lagervorrichtung 3 jedoch mehr als zwei Lagerschächte 5, und diese sind um eine vertikale Achse drehbar in einem Karussell angeordnet. Eine entsprechende Vorrichtung ist z.B. in WO 02/059251 beschrieben. Das Karussell in der Ausführung nach Fig. 3 wird vom Schachtträger 8 gebildet.

Bei dieser Ausführung ist der Schüttelantrieb 4 dazu ausgestaltet, den Schachtträger 8 nicht nur in vertikaler Richtung zu schütteln, sondern auch um seine vertikale Mittelachse 22 zu drehen, d.h. beide Bewegungen werden von nur einem Motor 20 erzeugt.

Auch in dieser Ausführung ruhen die Lagerschächte 5 auf einem Schachtträger 8, welcher in vertikaler, nicht aber in horizontaler Richtung auslenkbar ist. Zudem ist er um die vertikale Mittelachse 22 des Karussells drehbar. Hierzu ist er in einem nicht genauer dargestellten Drehlager 23 gehalten, welches z.B. als Zylinderbüchse in der Mittelachse 22 ausgestaltet sein kann.

Der Schachtträger 8 ruht auf drei Rollen 23, welche symmetrisch um die Mittelachse 22 des Karussells angeordnet sind. Jede Rolle ist exzentrisch auf einer horizontalen, radial zur Mittelachse 22 stehenden Rollenachse 24 gelagert. Jede Rollenachse 24 ist im stationären Grundkörper 10 gelagert und trägt konzentrisch ein erstes Kegelrad 26, welches zusammen mit einem zweiten Kegelrad 27 ein Winkelgetriebe bildet. Die zweiten Kegelräder 27 sind über einen Zahnriemen 19 und einen Motor 20 synchron angetrieben.

Wird der Zahnriemen 19 in Betrieb gesetzt, so beginnen sich die Rollen 23 exzentrisch um die Rollenachsen 24 zu drehen und versetzen den Schachtträger 8 in eine im wesentlichen konstante Drehbewegung und gleichzeitig in eine alternierende vertikale Auf- und Abbewegung, so dass die Lagerschächte 5 vertikal geschüttelt werden.

In einer Variante zu diesem Ausführungsbeispiel ist lediglich eine der Rollen 23 angetrieben.

Durch die in Fig. 3 gezeigte Ausgestaltung wird es möglich, das Karussell der Lagerschächte 5 mit nur einem Antrieb zu drehen und zu schütteln.

Es ist jedoch auch denkbar, einen anderen vertikalen Schüttelantrieb, z.B. einen Schüttelantrieb mit Taumellagern gemäss Fig. 1, mit einem konventionellen Karussellantrieb zu kombinieren. In diesem Falle verfügt die Vorrichtung also über einen Drehantrieb zum Drehen der Lagerschächte um die vertikale Achse 22 und einen Schüttelantrieb zum Erzeugen einer Auf- und Abbewegung der Lagerschächte. Dies ist konstruktiv etwas aufwändiger, erlaubt es aber, die Schüttelfrequenz unabhängig von der Drehgeschwindigkeit zu wählen.

Damit die Proben in handelsüblichen Mikrotiterplatten ausreichend durchmischt werden, sollte in den bisher beschriebenen Ausführungen die vertikale Schüttelbewegung eine Höhenamplitude zwischen 0.1 und 10 mm haben.

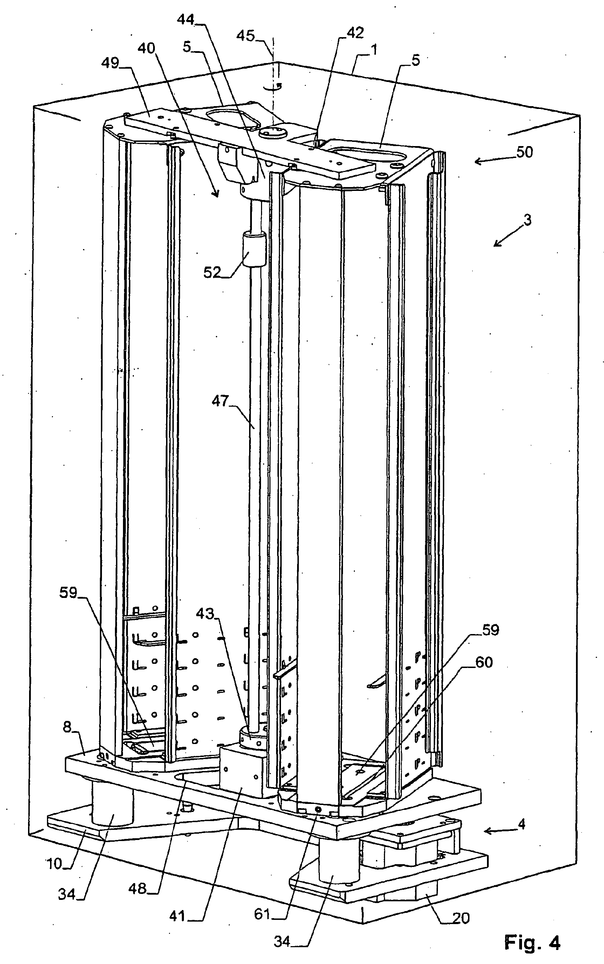

Gemäss der vorliegenden Erfindung werden die Lagerschächte in horizontaler Richtung geschüttelt. Eine entsprechende Ausführung ist in Fig. 4 bis 7 dargestellt.

Auch hier handelt es sich um eine Lagervorrichtung 3 mit zwei V-förmig angeordneten Lagerschächten 5, die z.B. in einem (in Fig. 4 angedeuteten) Klimaschrank 1 angeordnet sein können. Die Lagervorrichtung 3 kann aber wiederum auch ausserhalb eines Klimaschranks zum Einsatz kommen. Zudem kann die Zahl der Lagerschächte 5 den jeweiligen Anforderungen angepasst werden, und die Erfindung kann auch für karussellförmige Anordnungen der Lagerschächte, oder auch für nur einen einzigen Lagerschacht, eingesetzt werden.

Die beiden Lagerschächte 5 sind, wie in Fig. 1, auf einem Schachtträger 8, der von einem Schüttelantrieb 4 bewegt wird.

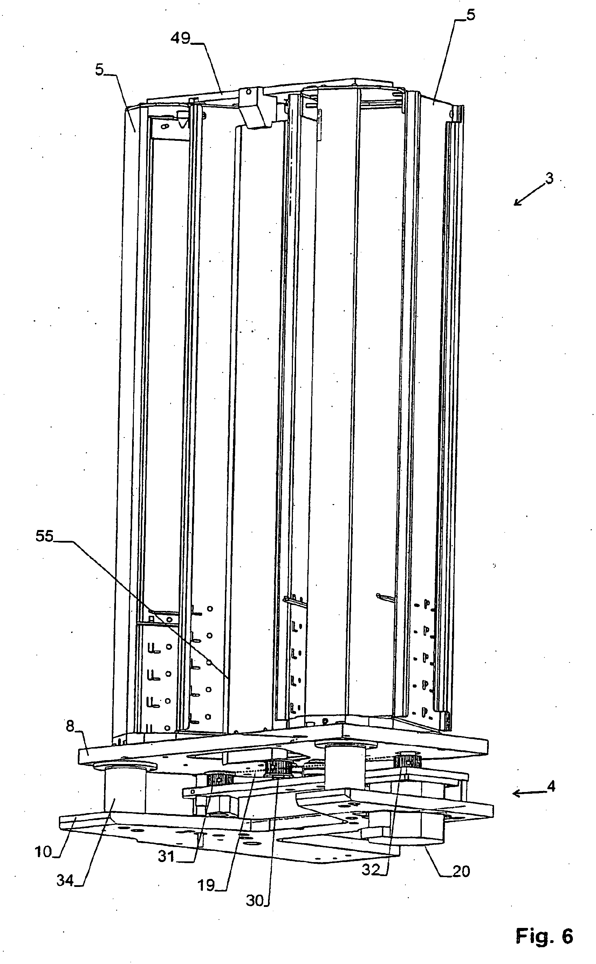

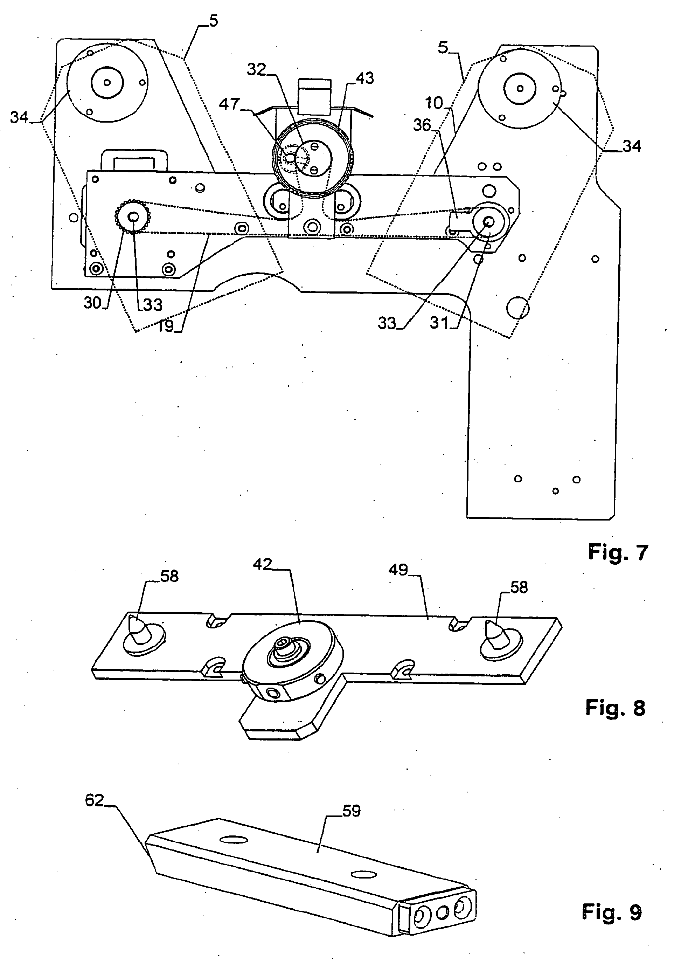

Im Gegensatz zur Ausführung nach Fig. 1 erzeugt der Schüttelantrieb nach Fig. 4 - 7 jedoch eine horizontale Schüttelbewegung, oder zumindest eine Schüttelbewegung mit horizontaler Bewegungskomponente. Hierzu ist ein Motor 20 vorgesehen, der direkt oder über einen in Fig. 6 und 7 gestrichelt dargestellten Zahnriemen 19 drei Ritzel 30, 31, 32 antreibt. Der Motor 20 und die Ritzel 30, 31, 32 sind am ruhenden Grundkörper 10 angeordnet.

Auf den zwei äusseren Ritzeln 30, 31 sind exzentrisch nach oben ragende Stifte 33 befestigt (siehe Fig. 7), die in entsprechende Öffnungen des Schachtträgers 8 eingreifen, und die als Exzenter wirken. Wenn sich die Ritzel 30, 31 drehen, wird somit über die Stifte 33 eine exzentrische horizontale Schüttelbewegung des Schachtträgers 8 erzeugt.

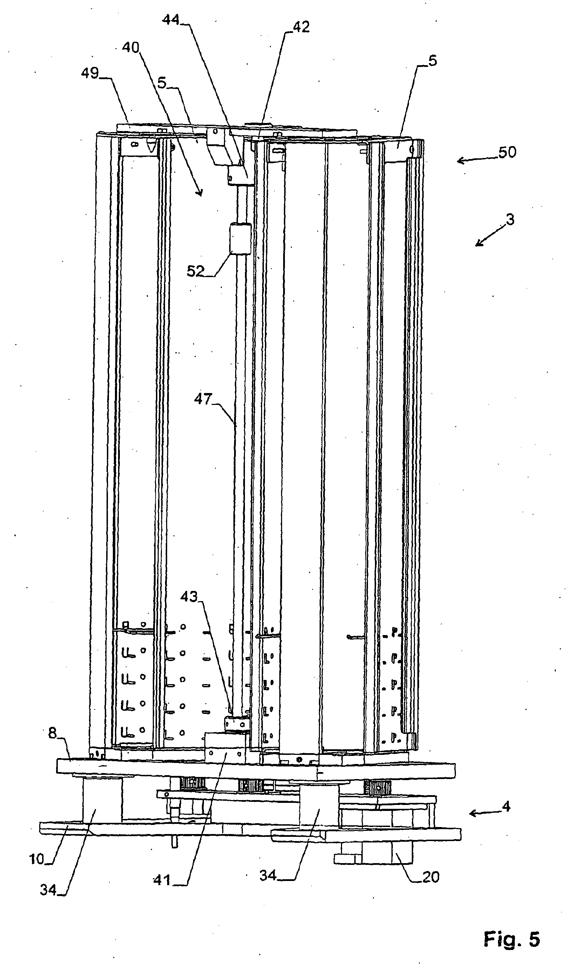

Dabei wird der Schachtträger von den Stiften 33 sowie von zwei Gleitlagern 34 getragen, und zwar so, dass er sich in horizontaler Richtung verschieben, nicht aber kippen kann. Eine zusätzliche vertikale Schüttelbewegung erfolgt in der Ausführung nach Fig. 5 - 7 nicht, wäre aber denkbar.

Wie aus Fig. 7 ersichtlich, ist an einem der Ritzel ein Arm 36 angeordnet, dessen Position von einem Messfühler erfasst werden kann. Dies erlaubt es, die absolute Position des Schachtträgers 8 zu bestimmen, so dass er bei Bedarf (z.B. für den Zugriff durch ein automatisches Bediensystem) in eine definierte Stellung gebracht werden kann.

Um trotz Schüttelbewegung die seitlichen Auslenkungen der Lagerschächte insbesondere in deren oberen Endbereich gering zu halten, ist in der Ausführung nach Fig. 4 - 7 zwischen den beiden Lagerschächten 5 eine Gegengewicht-Anordnung 40 vorgesehen. Diese umfasst einen bewegten Teil, der zwischen einem unteren Auslenklager 41 und einem oberen Auslenklager 42 angeordnet ist. Das untere Auslenklager 41 befindet sich auf der Höhe des Schachtträgers 8 und erstreckt sich, im Hinblick auf einen kompakten aber stabilen Aufbau, durch eine Öffnung oder Ausnehmung 48 desselben. Es ist am Grundkörper 10 montiert. Das obere Auslenklager 42 befindet sich auf der Höhe des oberen Endbereichs 50 der Lagerschächte und ist an einer Abstützung 49 befestigt, welche ihrerseits fest aber lösbar auf dem oberen Ende beider Lagerschächte 5 aufliegt.

Fig. 8 zeigt die Abstützung 49 von unten. Ersichtlich sind zwei Dorne 58, welche lösbar in die Lagerschächte 5 eingreifen, sowie das obere Auslenklager 42.

In beiden Auslenklagern 41, 42 (siehe Fig. 4) ist je ein Drehtisch 43, 44 drehbar um eine vertikale Gegengewicht-Drehachse 45 gehalten. Der untere Drehtisch 43 wird über das Ritzel 32 vom Zahnriemen 19 angetrieben, und zwar mit gleicher Drehzahl und synchron zu den Ritzeln 30 und 31. Der obere Drehtisch 44 ist nicht angetrieben aber frei um die Gegengewicht-Drehachse 45 drehbar.

Zwischen den beiden Drehtischen 43, 44 erstreckt sich von der Höhe des Schachtträgers 8 auf die Höhe des oberen Endbereichs 50 der Lagerschächte 5 ein stangenförmiger Gegengewicht-Träger 47. Dieser verläuft vertikal und ist exzentrisch zur Gegengewicht-Drehachse 45 angeordnet. Am Gegengewicht-Träger 47 ist ein Gegengewicht 52 befestigt, und zwar auf einer Höhe zwischen dem Schachtträger 8 und dem oberen Endbereich 50 der Lagerschächte 5. Vorzugsweise ist das Gegengewicht 52 entlang dem Gegengewicht-Träger 47 verschiebbar und kann auf der gewünschten Höhe arretiert werden.

Wie erwähnt, werden die Ritzel 30, 31, 32 synchron über den Zahnriemen 19 angetrieben und besitzen die gleiche Drehgeschwindigkeit (d.h. Umdrehungsrate). Somit besteht also zwischen der Position der exzentrischen Stifte 33 und jener des unteren Drehtisches 43 eine feste Phasenbeziehung. Diese ist vorzugsweise so gewählt, dass zwischen den Positionen der Stifte 33 keine Phasenverschiebung besteht, und zwischen jener der Stifte und jener des unteren Drehtisches 43 (bzw. des darauf montierten Gegengewicht-Trägers 47) eine Phasenverschiebung von 180°. Wird nun der Motor 20 in Betrieb gesetzt, so wird der Gegengewicht-Träger 47 gegenläufig zum Schachtträger 8 ausgelenkt, so dass er dem durch die Schüttelbewegung erzeugten Schwingen der Lagerschächte entgegenwirkt.

Dabei ist es vorteilhaft, wenn die Masse des bewegten Teils der Gegengewicht-Anordnung 40 multipliziert mit der Auslenkamplitude des bewegten Teils ungefähr gleich der Masse des aller Lagerschächte 5 und des Schachtträgers 8 multipliziert mit der Auslenkamplitude des Schachtträgers 8 ist. Wie insbesondere aus Fig. 7 ersichtlich, ist in der dargestellten Ausführung die Auslenkamplitude des bewegten Teils der Gegengewicht-Anordnung 40 wesentlich grösser als jene der Stifte 33 und somit des Schachtträgers 8, weshalb die Masse des bewegten Teils der Gegengewicht-Anordnung 40 relativ gering bleiben kann.

Die Gegengewicht-Anordnung 40 kann mit einer Schutzplatte 55 abgedeckt sein, wie sie in Fig. 6 eingezeichnet ist.

Um Platz zu sparen, befindet sich die Gegengewicht-Anordnung 40 in der dargestellten Ausführung zwischen den beiden V-förmig angeordneten Lagerschächten 5. Bei einer sternförmigen bzw. karussellförmigen Anordnung in der Art von Fig. 3 kann die Gegengewicht-Anordnung 40 in der Mitte des Karussells untergebracht werden.

Im Hinblick auf die Schüttelbewegung aller der hier beschriebenen Ausführungen sollten die Lagerschächte 5 gut am Schachtträger 8 befestigt werden. Hierzu ist für jeden Lagerschacht 5 eine Halteschiene 59 auf dem Schachtträger 8 angeordnet, welche in Fig. 4 ersichtlich und in Fig. 9 einzeln dargestellt ist. Die Halteschienen 59 werden in Ausnehmungen 60 an den Füssen der Lagerschächte 5 aufgenommen und durch horizontale Gewindestifte 61 zentriert und gehalten (siehe Fig. 4), mit denen die Lagerschächte 5 an den Halteschienen 59 festgeklemmt werden können. Die Halteschiene 59 - und dazu korrespondierend die Ausnehmungen 60 an den Füssen der Lagerschächte 5 - weist auf der dem Gewindestift 61 gegenüber liegenden Seite eine Neigung/Schräge 62 (siehe Fig. 9) zur Zentrierung und Fixierung der Lagerschächte 5 auf dem Schachtträger 8 auf.

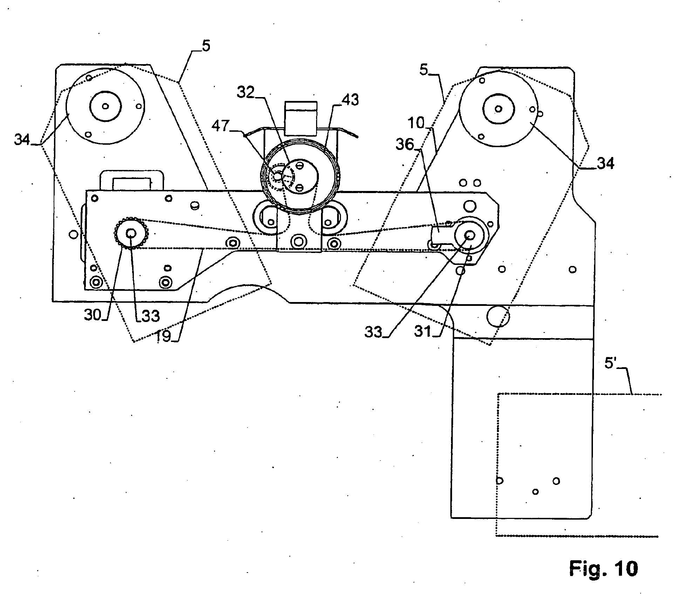

In den soweit gezeigten Ausführungsbeispielen werden jeweils alle Lagerschächte geschüttelt. Es ist jedoch auch denkbar, nur einen Teil der Lagerschächte zu schütteln, während ein anderer Teil auch bei eingeschaltetem Schüttelantrieb in Ruhe bleibt. Ein entsprechendes Beispiel ist in Fig. 10 illustriert. Es zeigt eine Vorrichtung, die an sich gleich aufgebaut ist wie jene nach Fig. 7, auf der jedoch ein zusätzlicher dritter Lagerschacht 5' vorgesehen ist. Dieser ruht nicht auf dem geschüttelten Schachtträger 8, sondern auf dem ruhenden Grundkörper 10. Somit befindet sich der dritte Lagerschacht 5' auch bei aktiviertem Schüttelantrieb 4 in Ruhe.

Es ist auch denkbar, zwei Schachtträger vorzusehen, welche wahlweise geschüttelt werden können, sei es über zwei separate Schüttelantriebe oder über wählbare Verbindung der beiden Schachtträger mit einem gemeinsamen Schüttelantrieb.

## Patentansprüche

1. Lagervorrichtung für Laborproben mit
einem Schüttelantrieb (4),
einem vom Schüttelantrieb (4) zu einer Schüttelbewegung mit horizontaler Komponente angetriebenen Schachtträger (8) und
mindestens einem auf dem Schachtträger (8) angeordneten Lagerschacht (5), welcher Lagerplätze für eine Mehrzahl von Laborproben übereinander aufweist,
**gekennzeichnet durch**
eine Gegengewicht-Anordnung (40), die mit einem oberen Endbereich des Lagerschachts (5) verbunden ist und angetrieben ist, um auf den oberen Endbereich eine zur Schüttelbewegung gegenläufige Kraft auszuüben um so einem **durch** die Schüttelbewegung erzeugten Schwingen des oberen Endbereichs des Lagerschachts (5) entgegenzuwirken.

2. Lagervorrichtung nach Anspruch 1, wobei die Gegengewicht-Anordnung (40) einen bewegten Teil (47, 52) aufweist, welcher gegenläufig zur Schüttelbewegung auslenkend angetrieben ist.

3. Schüttelvorrichtung nach Anspruch 2, wobei der bewegte Teil (47, 52) ein Gegengewicht (52) aufweist, welches vertikal verschiebbar ist.

4. Lagervorrichtung nach einem der Ansprüche 2 oder 3, wobei der bewegte Teil (47, 52) auslenkbar zwischen einem oberen und einem unteren Auslenklager (41, 42) angeordnet ist, wobei das obere Auslenklager (41, 42) am oberen Endbereich (50) des Lagerschachts (5) abgestützt ist.

5. Lagervorrichtung nach Anspruch 4, wobei am oberen und am unteren Auslenklager (41, 42) ein Drehtisch (43, 44) vorgesehen ist, in welchen der bewegte Teil (47, 52) exzentrisch gelagert ist.

6. Lagervorrichtung nach Anspruch 5, wobei der Schachtträger (8) von mindestens einem Exzenter (33) ausgelenkt ist, wobei der Exzenter (33) und der Drehtisch (43) am unteren Auslenklager (41) synchron und mit gleicher Drehgeschwindigkeit angetrieben sind, insbesondere über einen gemeinsamen Antriebsriemen.

7. Lagervorrichtung nach einem der Ansprüche 4 bis 6, wobei das obere Auslenklager (42) eine Abstützung (49) aufweist, welche an einem oberen Ende des Lagerschachts (5) befestigt ist.

8. Lagervorrichtung nach einem der Ansprüche 2 bis 7, wobei die Masse des bewegten Teils (47, 52) der Gegengewicht-Anordnung (40) multipliziert mit der Auslenkamplitude des bewegten Teils (47, 52) ungefähr gleich der Masse des Lagerschachts (5) bzw. der Lagerschächte und des Schachtträgers (8) multipliziert mit der Auslenkamplitude des Schachtträgers (8) ist.

9. Lagervorrichtung nach einem der Ansprüche 2 bis 8, wobei die Auslenkung des bewegten Teils (47, 52) grösser ist als die Auslenkung des Schachtträgers (8).

10. Lagervorrichtung nach einem der vorangehenden Ansprüche, wobei sie mindestens zwei Lagerschächte (5) aufweist und wobei die Gegengewicht-Anordnung (40) zwischen den Lagerschächten (5) angeordnet ist.

11. Lagervorrichtung nach Anspruch 10, wobei das obere Auslenklager (41, 42) eine Abstützung (49) aufweist, welche am oberen Ende aller Lagerschächte (5) befestigt ist.

12. Lagervorrichtung nach einem der vorangehenden Ansprüche, wobei sich die Gegengewicht-Anordnung (40) durch eine Öffnung oder Ausnehmung (48) des Schachtträgers (8) erstreckt.

13. Lagervorrichtung nach einem der vorangehenden Ansprüche, wobei die Gegengewicht-Anordnung (40) einen Gegengewicht-Träger (47) aufweist, welcher sich von einer Höhe des Schachtträgers (8) bis zu einer Höhe des oberen Endbereichs (50) erstreckt.

14. Lagervorrichtung nach Anspruch 13, wobei der Gegengewicht-Träger (47) gegenläufig zur Schüttelbewegung ausgelenkt ist, und insbesondere wobei am Gegengewicht-Träger (47) zwischen der Höhe des Schachtträgers (8) und der Höhe des oberen Endbereichs ein Gegengewicht (52) angeordnet ist.

15. Lagervorrichtung nach einem der vorangehenden Ansprüche, wobei die Lagerschächte (5) ausgestaltet sind für die Aufnahme von Mikrotiterplatten.

16. Lagervorrichtung nach einem der vorangehenden Ansprüche, wobei wahlweise oder dauernd nur ein Teil (5) der Lagerschächte vom Schüttelantrieb (4) schüttelbar sind, während mindestens ein weiterer Lagerschacht (5') in Ruhe bleibt.

17. Lagervorrichtung nach einem der vorangehenden Ansprüche, wobei die Lagerschächte in einem Karussell angeordnet sind, welches mit einem Drehantrieb um eine vertikale Achse (22) drehbar ist.

18. Klimaschrank mit einer Lagervorrichtung (3) nach einem der vorangehenden Ansprüche.

## Claims

1. A storage device for laboratory samples comprising
a shaker drive (4),
a rack carrier (8) being driven by said shaker drive (4) for performing a shaking motion with horizontal component, and
at least one storage rack (5) arranged on the rack carrier (8), which comprises storage locations for a plurality of laboratory samples on top of each other,
**characterized by**
a counterweight assembly (40) connected to an upper end section of the storage rack (5) and being driven to exert, to the upper end section, a force opposite to the shaking motion in order to counteract an oscillation, caused by the shaking motion, of the upper end section of the storage rack (5).

2. The storage device of claim 1 wherein the counterweight assembly (40) comprises a moving part (47, 52) driven to move opposite to the shaking motion.

3. The storage device of claim 2 wherein the moving part (47, 52) comprises a vertically displaceable counterweight (52).

4. The storage device of any of the claims 2 or 3 wherein the moving part (47, 52) is moveable between an upper and a lower support (41, 42), wherein the upper support (41, 42) is braced against the upper end section (50) of the of the storage rack (5).

5. The storage device of claim 4 further comprising rotating tables (43, 44) mounted to the upper and lower supports (41, 42) in which said moving part (47, 52) is mounted eccentrically.

6. The storage device of claim 5 wherein the rack carrier (8) is moved by at least one eccentric member (33), wherein the eccentric member (33) and the rotating table (43) are driven, at the lower support (41), synchronously and with equal rotational speed, in particular by means of a common driving belt.

7. The storage device of any of the claims 4 to 6, wherein the upper support (42) comprises a strut (49) connected to an upper end of the storage rack (5).

8. The storage device of any of the claims 2 to 7, wherein the mass of the moving part (47, 52) of the counterweight assembly (40) multiplied by the amplitude of motion of the moving part (47, 52) is approximately equal to the mass of the storage rack (5) or storage racks and the rack carrier (8) multiplied by the amplitude of motion of the rack carrier (8).

9. The storage device of any of the claims 2 to 8, wherein the amplitude of motion of the moving part (47, 52) is larger than the amplitude of motion of the rack carrier (8).

10. The storage device of any of the preceding claims, wherein it comprises at least two storage racks (5) and wherein the counterweight assembly (40) is arranged between the storage racks (5).

11. The storage device of claim 10 wherein the upper support (41, 42) comprises a strut (49) connected to the upper end of all storage racks (5).

12. The storage device of any of the preceding claims, wherein the counterweight assembly (40) extends through an opening or recess (48) of the rack carrier (8).

13. The storage device of any of the preceding claims, wherein the counterweight assembly (40) comprises a counterweight carrier (47) extending from a height of the rack carrier (8) to a height of the upper end section (50).

14. The storage device of claim 13, wherein the counterweight carrier (47) is driven to move opposite to the shaking motion, and in particular wherein a counterweight (52) is mounted on the counterweight carrier (47) between the height of the rack carrier (8) and the height of the upper end section.

15. The storage device of any of the preceding claims, wherein the storage racks (5) are adapted to receive micro titer plates.

16. The storage device of any of the preceding claims wherein, selectively or continuously, it is possible to shake only a part (5) or the storage racks by means of the shaker drive (4) while at least one further storage rack (5') remains at rest.

17. The storage device of any of the preceding claims, wherein the storage racks are arranged in a carousel, which is rotatable about a vertical axis (22) by means of a rotating drive.

18. A climate controlled cabinet with a storage device (3) of any of the preceding claims.

## Revendications

1. Dispositif de stockage pour échantillons de laboratoire comportant
un moteur d'agitation (4),
un support de puits (8) entraîné, par le moteur d'agitation (4), en un mouvement d'agitation avec composante horizontale et
au moins un puits de stockage (5) disposé sur le support de puits (8), lequel présente des emplacements de stockage pour une pluralité d'échantillons de laboratoire l'un au-dessus de l'autre,
**caractérisé par**
un dispositif à contrepoids (40) qui est relié à une zone terminale supérieure du puits de stockage (5) et est entraîné afin d'exercer, sur la zone terminale supérieure, une force en sens contraire au mouvement d'agitation, afin de s'opposer ainsi à une oscillation, produite par le mouvement d'agitation, de la zone terminale supérieure du puits de stockage (5).

2. Dispositif de stockage selon la revendication 1, dans lequel le dispositif à contrepoids (40) comporte une partie (47, 52) en mouvement qui est entraînée dans une excursion en sens contraire au mouvement d'agitation.

3. Dispositif de stockage selon la revendication 2, dans lequel la partie en mouvement (47, 52) comporte un contrepoids (52) qui peut coulisser verticalement.

4. Dispositif de stockage selon l'une quelconque des revendications 2 ou 3, dans lequel la partie en mouvement (47, 52) est disposée de manière à pouvoir être déviée entre un palier de déviation supérieur et inférieur (41, 42), le palier de déviation supérieur (41, 42) étant soutenu dans la zone terminale supérieure (50) du puits de stockage (5).

5. Dispositif de stockage selon la revendication 4, dans lequel il est prévu dans le palier de déviation supérieur et le palier de déviation inférieur (41, 42) une table rotative (43, 44) dans laquelle la partie en mouvement (47, 52) est supportée de manière excentrée.

6. Dispositif de stockage selon la revendication 5, dans lequel le support de puits (8) est dévié par au moins un excentrique (33), l'excentrique (33) et la table rotative (43) sur le palier de déviation inférieur (41), étant entraînés de manière synchrone et avec la même vitesse de rotation, en particulier par l'intermédiaire d'une courroie d'entraînement commune.

7. Dispositif de stockage selon l'une quelconque des revendications 4 à 6, dans lequel le palier de déviation supérieur (42) présente un appui (49) qui est fixé à une extrémité supérieure du puits de stockage (5).

8. Dispositif de stockage selon l'une quelconque des revendications 2 à 7, dans lequel la masse de la partie en mouvement (47, 52) du dispositif à contrepoids (40) multipliée par l'amplitude de déviation de la partie en mouvement (47, 52), est environ égale à la masse du puits de stockage (5) ou des puits de stockage et du support de puits (8), multipliée par l'amplitude de déviation du support de puits (8).

9. Dispositif de stockage selon l'une quelconque des revendications 2 à 8, dans lequel la déviation de la partie en mouvement (47, 52) est supérieure à la déviation du support de puits (8).

10. Dispositif de stockage selon l'une quelconque des revendications précédentes, qui comporte au moins deux puits de stockage (5) et dans lequel le dispositif à contrepoids (40) est disposé entre les puits de stockage (5).

11. Dispositif de stockage selon la revendication 10, dans lequel le palier de déviation supérieur (41, 42) présente un appui (49) qui est fixé à l'extrémité supérieure de tous les puits de stockage (5).

12. Dispositif de stockage selon l'une quelconque des revendications précédentes, dans lequel le dispositif à contrepoids (40) s'étend à travers une ouverture ou évidement (48) du support de puits (8).

13. Dispositif de stockage selon l'une quelconque des revendications précédentes, dans lequel le dispositif à contrepoids (40) comporte un support de contrepoids (47) qui s'étend d'une hauteur du support de puits (8) à une hauteur de la zone d'extrémité supérieure (50).

14. Dispositif de stockage selon la revendication 13, dans lequel le support de contrepoids (47) est dévié en sens contraire au mouvement d'agitation, et dans lequel en particulier un contrepoids (52) est disposé sur le support de contrepoids (47), entre la hauteur du support de puits (8) et la hauteur de la zone d'extrémité supérieure.

15. Dispositif de stockage selon l'une quelconque des revendications précédentes, dans lequel les puits de stockage (5) sont conçus pour recevoir des plaques de microtitrage.

16. Dispositif de stockage selon l'une quelconque des revendications précédentes, dans lequel, au choix ou en permanence, seule une partie (5) des puits de stockage peut être agitée par le moteur d'agitation (4), tandis qu'au moins un autre puits de stockage (5') reste au repos.

17. Dispositif de stockage selon l'une quelconque des revendications précédentes, dans lequel les puits de stockage sont disposés dans un carrousel qui peut tourner, avec un dispositif d'entraînement en rotation, autour d'un axe vertical (22).

18. Armoire climatique avec un dispositif de stockage (3) selon l'une quelconque des revendications précédentes.
